Europäisches Patentamt

European Patent Office

Office européen des brevets

(19)

(11) Veröffentlichungsnummer: **0 162 007**
**A1**

(12) ## EUROPÄISCHE PATENTANMELDUNG

(21) Anmeldenummer: 85810165.2

(22) Anmeldetag: 15.04.85

(51) Int. Cl.⁴: **A 61 K 37/26,** A 61 K 47/00

(30) Priorität: 18.04.84 CH 1996/84

(43) Veröffentlichungstag der Anmeldung: **21.11.85**
**Patentblatt 85/47**

(84) Benannte Vertragsstaaten: **AT BE CH DE FR GB IT LI NL**
**SE**

(71) Anmelder: **Geriaco AG, Pfeffingerstrasse 41,**
**CH-4053 Basel (CH)**

(72) Erfinder: **Dahlgren, Stig Ake, Fregattvägen 9,**
**S-181 37 Lidingö (SE)**

(74) Vertreter: **Frossard, Michel, Dr. et al, A. Braun, Braun,**
**Héritier, Eschmann AG, Patentanwälte**
**Holbeinstrasse 36-38, CH-4051 Basel (CH)**

(54) **Stabiles, lagerungsfähiges Insulinpräparat zur Behandlung von Akne vulgaris.**

(57) Die Akne vulgaris kann durch äusserliche Anwendung von Insulin in Form eines Gels erfolgreich behandelt werden; ein stabiles, lagerungsfähiges Gel entsteht durch Verwendung einer Polyacrylsäure, deren Carboxylgruppen neutralisiert sind. Durch Kombination des Insulins mit dem Enzym Hyaluronidase oder/und α-Glucosidase kann eine Steigerung der Wirkung erreicht werden. Das Präparat kann sowohl kurativ als auch prophylaktisch und als Dauerbehandlung verwendet werden, um einen erneuten Ausbruch der Krankheit zu verhindern.

ACTORUM AG

0162007

Stabiles, lagerungsfähiges Insulinpräparat zur Behandlung der Akne vulgaris

Die Akne vulgaris ist eine inflammatorische Störung, welche häufig und ziemlich allgemein in den mittleren und späteren Jugendjahren auftritt. Sie kommt bei allen Rassen vor, zeigt aber einen gelinderen Verlauf bei gewissen orientalen Völkern. Man schätzt, dass ca. 60 % der Jugendlichen von der Pubertät an bis etwa zum 25. Lebensjahr davon betroffen werden; nach einer anderen Schätzung leiden etwa 100 Millionen Jugendliche darunter in der westlichen Welt (Westeuropa und Nordamerika).

Die Akne zeigt sich klinisch durch das Auftreten von Komedonen, Papulen, Knötchen und Bläschen, die sich in charakteristischer Weise verteilen, nämlich auf der fetten Gesichtshaut, mit Ausnahme des periorbitalen Bereiches, auf dem Nacken, der Brust und den Schultern. Die Schäden bei der Akne werden in solche inflammatorischen und nicht inflammatorischen Typus eingeteilt; nicht inflammatorische Schäden enthalten offene und geschlossene Komedonen. Es werden beide Geschlechter betroffen, bei den Männern sind die Schäden in manchen Fällen schwerer.

Zur Behandlung der Akne vulgaris sind schon zahlreiche Produkte vorgeschlagen und versucht worden, insbesondere verschiedene Hautwaschmittel, das Natriumborat, das Natriumlaurylsulfat, das Benzoylperoxid, die Retinoesäure (Tretinoin, Vitamin-A-säure) und Antibiotika, wie das Neomycin und das Tetracyclin-hydrochlorid. Sämtliche bisher geprüfte Produkte bewirken
allenfalls eine temporäre Besserung, die Wirkung nimmt
jedoch nach einiger Zeit ab und es treten mitunter Nebenwirkungen auf, welche manchmal so schwerwiegend wie
die Akne selbst sein können.

So kann das Benzoylperoxid (US Patent Nr. 4 387 107) Hautreizungen, die bestehen bleiben, verursachen, ferner Hautätzungen und
Narbenbildungen, welche zu schweren Dermatosen führen.
Eine zwar sekundäre, aber dennoch lästige Nebenwirkung
des Benzoylperoxids ist seine Fähigkeit zum Bleichen von
Textilien, d.h. von Kleidungsstücken. Auch die Retinoesäure kann intensive Hautreizungen bewirken; bei der
Behandlung mit diesem Produkt war es zudem schwierig,
eindeutige Besserungen festzustellen. Das Neomycin
zeigt in der Infektionsphase der Akne relativ gute Ergebnisse; nach kurzer Dauer der Behandlung entwickelt
sich allerdings eine Resistenz, so dass damit auf langer
Sicht keine oder eine nur geringe Besserung erreicht
wird. Das Tetracyclin seinerseits kann bei einer Langzeitbehandlung eine mildernde Wirkung entfalten, es erzeugt aber bekanntlich eine Anzahl Nebenwirkungen, wie
Diarrhoe, Müdigkeit und Kopfschmerzen.

Insgesamt hat sich bisher keines der untersuchten Produkte bei der Behandlung der Krankheit allgemein bewährt: die anfänglich günstige Wirkung gewisser Produkte war nicht von Dauer oder es traten Nebenwirkungen auf, welche zum Absetzen der Behandlung
zwangen. Vorübergehende Erfolge bzw. Nebenwirkungen können jedoch umso weniger hingenommen werden, als die Akne
eine Dauerbehandlung, öfters sogar während Jahre, erfordert.

Andererseits haben ältere Veröffentlichungen von einer gewissen Besserung im Zustand von nicht diabetischen Patienten berichtet, welche an Akne litten und einer Insulinbehandlung unterworfen wurden. Die Behandlung bestand aus einer dauerhaften Serie von Insulinschocks bei 6 psychotischen, schizophrenen Patienten [J. Wortis, J. Am. Med. Assoc. 108, 971 (1937)], einer Serie von parenteralen Insulininjektionen zwei- bis dreimal pro Woche bei 13 weiteren Patienten [H.C. Semon und F. Herrmann, Brit. J. Derm. 52, 123 (1940)] bzw. der Injektion von Insulin direkt in die Akne-Pusteln (Papulae) bei einigen wegen Geistesstörung hospitalisierten Jugendlichen [R.W. Grover und N. Arikian, J. Invest. Derm. 40, 259 (1963)].

Dass eine Insulinschock-Therapie oder Insulininjektionen zur Dauerbehandlung der Akne bei ansonsten gesunden Jugendlichen überhaupt nicht in Frage kommen, dürfte an und für sich klar sein. Deshalb sind die oben erwähnten Berichte auch bald in Vergessenheit geraten und haben zu keinen weiteren Untersuchungen Anlass gegeben. Auch die Furcht vor einer hypoglykämischen Reaktion als Folge der systemischen Wirkungen des Insulins, wie von Grover und Arikian am Schluss ihres Berichtes hervorgehoben, liess von weiteren Versuchen absehen.

Um so überraschender war es nun, als gefunden wurde, dass das Insulin in einer ad hoc geschaffenen pharmazeutischen Form bei äusserlicher Anwendung auf der Haut von an der Akne vulgaris leidenden Jugendlichen die Krankheit zu heilen oder zumindest deren Verlauf günstig zu beeinflussen vermag, ohne dabei jegliche Nebenwirkungen und namentlich ohne systemische Wirkungen zu entfalten. Die Besserung tritt nach kurzer Zeit ein: die Entzündungen und die Anschwellungen gehen zurück, die Rötung der betroffenen Körperteile nimmt ab und mit dem Abklingen der Infektion wird die Haut normalisiert. Eingeschlossene Komedonen können wieder aufflammen, werden aber durch eine erneute Behandlung leicht geheilt.

Die Wirkung des Insulins muss sich nicht auf eine kurative Behandlung der an Akne bereits leidenden Jugendlichen beschränken, das Insulin kann auch mit Erfolg prophylaktisch angewendet werden. Je früher nämlich eine aktive Behandlung der Akne begonnen wird, desto besser sind die Ergebnisse und desto weniger Beschwerden treten auf. Bei Veranlagung und jedenfalls bei den geringsten Anzeichen von Akne sollte die Behandlung eingesetzt und, um der Infektion entgegenzuwirken und die Krankheit unter Kontrolle zu halten, fortgesetzt werden.

Einem stabilen, lagerungsfähigen Insulinpräparat stellen sich aber spezifische Beschränkungen bzw. Erfordernisse entgegen, welche im Falle der Akne vulgaris durch die Beschaffenheit der erkrankten Haut selber bedingt sind. So dürfen als Trägermaterial oder Exzipiens keine Fettstoffe oder fettende Produkte verwendet werden; damit scheiden die herkömmlichen Salbengrundlagen von vornherein aus.

Zudem stellt das Insulin bekanntlich eine besonders empfindliche chemische Verbindung dar, welche durch die verschiedensten äusseren Einflüsse leicht zersetzt wird. Beispielsweise kann die Berührung mit Glas schon genügen, um eine langsame Zersetzung der Molekel einzuleiten. Auch die Temperatur kann sich bei langer Lagerung eines Insulinpräparates negativ auf die Stabilität auswirken. So müssen Insulinlösungen, wenn sie nicht genau bei neutralem pH-Wert eingestellt sind, im Kühlschrank aufbewahrt werden; ansonsten lässt sich bei Temperaturen gegen 20°C bereits Zersetzung feststellen. Aehnlich verhält sich die Molekel gegenüber den meisten chemischen Substanzen.

Unter anderen hat die Verwendung von wasserlöslichen Celluloseethern als Geliermittel die Herstellung von Insulin enthaltenden Gelen ermöglicht, welche in bezug auf Konsistenz und Aussehen durchaus befriedigend waren. Bei der Prüfung auf Stabilität bzw. Lagerungsfähigkeit auf lange Dauer zeigte sich aber, dass das Insulin eine teilweise Zersetzung erlitten hatte. Dieselben negativen Erfahrungen mussten in der Folge mit anderen bekannten Geliermitteln gemacht werden.

Unerwarteterweise wurde jedoch in einer hochmolekularen Polyacrylsäure, deren Carboxylgruppen neutralisiert sind, ein für die angestrebten Zwecke ideales Geliermittel gefunden. Es lassen sich nämlich damit wasserhaltige, Insulin enthaltende Gele herstellen, welche sich nicht nur durch die gewünschte Konsistenz und ein befriedigendes Aussehen, sondern darüber hinaus auch durch eine hervorragende Lagerungsfähigkeit bzw. Stabilität der Insulinmolekel auszeichnen. Die nun über mehr als 6 Monate laufenden Stabilitätsversuche bei Raumtemperatur haben keine Anhaltspunkte für eine Zersetzung des Wirkstoffes geliefert.

Unter den Acrylsäure-Polymerisaten, auch Polyacrylsäuren oder Carboxyvinylpolymere genannt, kann man insbesondere solche verwenden, welche unter dem Markennamen Carbopol erhältlich sind, beispielsweise Carbopol 941, Carbopol 934 oder Carbopol 940 mit einem durchschnittlichen Molekulargewicht von 1'250'000, 3'000'000 und 4'000'000 resp. (Hersteller: B.F. Goodrich Chemical Group, Cleveland, OH/USA).

Die sauren Gruppen der Polyacrylsäure müssen durch eine wasserlösliche anorganische oder organische Base neutralisiert sein. Als anorganische Base eignen sich insbesondere Natronlauge und Kalilauge; vorzugsweise wird Natronlauge verwendet, einerseits wegen der physiologischen Rolle des Natriumions, andererseits infolge der leichten stoechiometrischen Dosierbarkeit von verdünnter Natronlauge. Als organische Base eignen sich wasserlösliche Amine. Ein solches Amin kann z.B. das Methylamin, das Dimethylamin, das Ethylamin, das Diethylamin, das Propylamin, das Isopropylamin, das Diisopropylamin, das Cyclohexylamin, das Benzylamin, das Guanidin, das Pyrrolidin, das Piperidin, das Morpholin das Arginin, das Lysin, das Ethanolamin, das Diethanolamin das Diisopropanolamin oder das Triethanolamin sein. Bevorzugt werden die bei Raumtemperatur flüssigen niederen aliphatischen Amine, beispielsweise das Diisopropanolamin oder das Diethylamin.

Der Gehalt des Gels an Insulin kann in einem weiten Bereich variieren, z.B. von etwa 0,25 bis 25 mg Insulin per ml Gel. Die Wirkung des Präparates auf die Akne lässt sich schon bei einem so minimalen Gehalt wie etwa 0,25 mg Insulin/ml Gel beobachten; bei höheren Konzentrationen, beispielsweise 0,50, 1, 2 oder 5 mg Insulin/ml Gel, steigt auch die Wirksamkeit der Behandlung. Vorzugsweise enthält das Gel 0,50 bis 5 mg Insulin/ml.

Das Gel soll einen physiologisch verträglichen pH-Wert aufweisen; es wird mit Vorteil auf einen pH-Bereich von 6,7 bis 7,2, vorzugsweise auf pH 6,8 bis 7,0 eingestellt. Zur Einstellung des pH-Wertes kann man die nötige Menge der oben erwähnten Base oder einer physiologisch verträglichen Säure, wie verdünnte Phosphorsäure, Salzsäure, Essigsäure, Milchsäure oder Brenztraubensäure, zugeben.

Es wurde ferner gefunden, dass die Zugabe des Enzyms Hyaluronidase oder/und des Enzyms $\alpha$-Glucosidase den Erfolg der Behandlung eindrücklich zu steigern vermag. Die Hyaluronidase erleichtert und verbessert das Eindringen des Gels in die Haut, während man sich von der $\alpha$-Glucosidase eine Unterstützung der lokalen Wirkung des Insulins auf den Kohlehydratstoffwechsel in der Haut vorstellen kann.

Dem Präparat kann ferner ein Konservierungsmittel zugegeben werden, wie z.B. das Nipagin® oder 4-Hydroxybenzoesäure-methylester bzw. -ethylester.

Die mit dem beschriebenen Gel unternommenen Untersuchungen an freiwilligen Aknepatienten beiderlei Geschlechtes laufen jetzt seit über 2 Jahren; es sind dabei keinerlei Nebenwirkungen beobachtet worden. Es konnten insbesondere bei keinem Patienten Anzeichen dafür festgestellt werden, dass das Insulin die Haut durchdringen und in die Blutbahn gelangen würde. Ueberhaupt ist in der reichlichen Literatur über das Insulin von einer möglichen perkutanen Absorption nichts zu finden; bekanntlich entfaltet die Verbindung ihre systemischen Wirkungen bei parenteraler, üblicherweise intramuskulärer, Verabreichung.

Mit der Behandlung der Akne soll möglichst frühzeitig, d.h. so weit wie möglich nach dem Auftreten der ersten Symptome, begonnen werden.

Im allgemeinen wird die Behandlung folgendermassen durchgeführt. Die angegriffenen Hautbereiche werden mit einem geeigneten Hautwaschmittel sorgfältig gereinigt und danach mit lauwarmem Wasser abgespült. Nach Abtrocknen der Haut wird das Insulingel in dünner Schicht auf die betroffenen Bereiche ausgestrichen. Auf schwerer geschwollene Teile kann zusätzliches Gel aufgetragen werden, das während der Nacht mit einem Pflaster bedeckt wird. In der Regel verschwinden solche Schwellungen und es tritt eine deutliche Besserung nach etwa 8 Stunden ein.

0162007

Als Beispiel sei der Fall eines 17-Jährigen erwähnt, welcher seit dem Alter von ungefähr 12 Jahren an einer ausgebreiteten Akne vulgaris im Gesicht, auf dem Rücken und auf den Schultern litt. Er war schon mit verschiedenen Produkten behandelt worden, jeweils mit nur geringem Erfolg. Nun wurde er während zwei Wochen täglich wie folgt behandelt: zuerst wurden die betroffenen Hautbereiche mit Seife und Wasser gewaschen, um von der Oberfläche der Haut überschüssiges Fett zu entfernen, dann wurde mit Wasser gründlich abgespült und sorgfältig abgetrocknet. Das Gel gemäss nachfolgendem Beispiel 1 (mit 0,50 mg Insulin per ml Gel) wurde auf diese Hautbereiche verteilt und schwer angegriffene Follikel wurden ausserdem mit einer dickeren Schicht Gel überzogen und danach mit einem Pflaster bedeckt. Die Besserung trat nach kurzer Zeit ein, die Rötung nahm langsam ab und mit dem Abklingen der Infektion erlangte die Haut ihr normales Aussehen wieder.

Die durch das Insulin bzw. das neue Präparat erzielten therapeutischen Erfolge sind von symptomatischer Natur; deshalb soll die Behandlung solange fortgesetzt werden, wie die Krankheit selbst weiterbesteht. Nun kann die Dauer einer unbeeinflusst, ohne jegliche Gegenmassnahmen verlaufenden Akne nicht im voraus abgeschätzt werden; sie beträgt einige Monate bis einige Jahre und ist ohnehin ganz individuell. Man wird also beispielsweise während 6 Monate täglich einmal behandeln, die Behandlung dann versuchsweise absetzen und nötigenfalls, d.h. bei erneutem Auftreten der Symptome, wieder einsetzen für eine weitere Zeitspanne.

Die lange Dauer der Behandlung, die sich bis zum endgültigen Abklingen der Akne öfters notwendig erweist, macht folgendes Behandlungsschema ratsam: es wird während mehrerer Monate, bis zum völligen Verschwinden der Symptome, täglich einmal mit etwa 0,25 bis 2 mg Insulin/ml Gel behandelt.

0162007

## Beispiel 1

- Man mischt 50 mg Insulin mit 10 ml entmineralisiertem Wasser unter Rühren                     Lösung_A

- Man mischt 1,5 g Carbopol®934 mit 20 ml
  entmineralisiertem Wasser, neutralisiert
  sorgfältig durch langsame Zugabe von verdünnter Natronlauge unter Rühren und
  rührt weiter bis zur Bildung einer homogenen Suspension, gibt langsam die Lösung A
  hinzu und rührt gründlich                                Gel_B

- Man lässt das Gel B über Nacht stehen

- Man gibt langsam unter gründlichem Rühren
  destilliertes Wasser bis auf 100,0 ml zu      Gel_C

## Beispiel 2

- Man mischt 2 g Carbopol®941 mit 30 ml
  entmineralisiertem Wasser, neutralisiert
  sorgfältig durch langsame Zugabe von verdünnter Natronlauge unter Rühren und stellt
  daraus durch fortgesetztes Rühren eine
  homogene Suspension her                                  Gel_A

- Man löst 500 mg Insulin in 20 ml entmineralisiertem Wasser unter Rühren                           Lösung_B

- Man gibt langsam die Lösung B zum Gel A
  und rührt gründlich                                      Gel_C

- Man lässt das Gel C über Nacht stehen und
  bringt es schliesslich auf ein Volumen von
  100 ml durch Zugabe von entmineralisiertem Wasser    Gel_D

## Beispiel 3

- Man mischt 3 g Carbopol® 940 mit 20 ml entmineralisiertem Wasser, neutralisiert sorgfältig durch
  langsame Zugabe einer Lösung von 3 g Diisopropanolamin in 10 ml entmineralisiertem Wasser unter Rühren
  und stellt daraus durch sorgfältiges Rühren eine
  homogene Suspension her                                  Gel_A

0162007

- Man löst 100 mg Insulin in 20 ml entmineralisiertem Wasser unter Rühren                                    Lösung_B

- Man löst 100 Internationale Einheiten
Hyaluronidase in 10 ml Wasser unter Rühren                       Lösung_C

- Man gibt langsam die Lösung C zur Lösung B
und rührt gründlich                                              Lösung_D

- Man gibt die Lösung D zum Gel A und
rührt gründlich                                                  Gel_E

- Man lässt das Gel E über Nacht stehen und
bringt es schliesslich auf ein Volumen von
100 ml durch Zugabe von entmineralisiertem Wasser   Gel_F

## Beispiel 4

- Man mischt 2 g Carbopol$^R$ 941 mit 20 ml entmineralisiertem Wasser, neutralisiert sorgfältig durch langsame Zugabe einer Lösung von 2 g Diethanolamin in
10 ml entmineralisiertem Wasser unter Rühren und
stellt daraus durch fortgesetztes Rühren eine homogene Suspension her                                             Gel_A

- Man löst 200 mg Insulin in 25 ml entmineralisiertem Wasser unter Rühren                                    Lösung_B

- Man gibt 5 ml Spritamylase SAN (flüssiges $\alpha$-Gluco-
sidase-Präparat der Firma Novo Industrie GmbH,
Mainz/BRD) in 5 ml Wasser unter Rühren                           Lösung_C

- Man gibt langsam die Lösung C zur Lösung B
und rührt gründlich                                              Lösung_D

- Man gibt die Lösung D langsam zum Gel A
und rührt gründlich                                              Gel_E

- Man lässt Gel E über Nacht stehen und bringt es
schliesslich auf ein Volumen von 100 ml durch Zugabe
von entmineralisiertem Wasser                                    Gel_F

Beispiele 5 bis 8

| Beispiel | 5 | 6 | 7 | 8 |
|---|---|---|---|---|
| Insulin (kristall.) | 40 mg | 80 mg | 120 mg | 160 mg |
| Zink (als $ZnCl_2$) | 2000 /ug | 4000 /ug | 6000 /ug | 8000 /ug |
| Protamin | 40 /ug | 80 /ug | 120 /ug | 160 /ug |
| 4-Hydroxibenzoe-säuremethylester | 25 mg | 50 mg | 75 mg | 100 mg |
| Carbopol(R) 934-Gel | 50 g | 50 g | 50 g | 50 g |

Natronlauge, quod sufficit ad pH 7,3

Man gibt die ersten vier obengenannten Komponenten langsam und unter ständigem Rühren zum Carbopolgel, rührt weiter bis zur Bildung eines homogenen Gels und bringt den pH-Wert durch Zugabe von verdünnter Natronlauge unter ständigem Rühren auf 7,3.

E

<u>P a t e n t a n s p r ü c h e</u>

1. Stabiles, lagerungsfähiges Insulinpräparat zur prophylaktischen und kurativen Behandlung der Akne vulgaris durch äusserliche Anwendung, dadurch gekennzeichnet, dass es aus einem waserhaltigen Gel besteht, welches - nebst Insulin selbst - als Geliermittel eine hochmolekulare Polyacrylsäure, deren Carboxylgruppen in Form eines Salzes mit einer wasserlöslichen anorganischen oder organischen Base vorliegen, enthält.

2. Insulinpräparat nach Anspruch 1, dadurch gekennzeichnet, dass als Polyacrylsäure eine solche mit einem Molekulargewicht von etwa 1 Million bis etwa 4 Millionen verwendet wird.

3. Insulinpräparat nach Anspruch 1 oder 2, dadurch gekennzeichnet, dass die Carboxylgruppen der Polyacrylsäure in Form des Natriumsalzes, des Ammoniumsalzes oder des Calciumsalzes vorliegen.

4. Insulinpräparat nach einem der Ansprüche 1 bis 3, dadurch gekennzeichnet, dass es 0,25 bis 25 mg Insulin per ml Gel enthält.

5. Insulinpräparat nach Anspruch 4, dadurch gekennzeichnet, dass es 0,50 bis 5 mg Insulin per ml Gel enthält.

6. Insulinpräparat nach einem der Ansprüche 1 bis 5, dadurch gekennzeichnet, dass es ausserdem Hyaluronidase enthält.

7. Insulinpräparat nach einem der Ansprüche 1 bis 5, dadurch gekennzeichnet, dass es ausserdem $\alpha$-Glucosidase enthält.

8. Insulinpräparat nach einem der Ansprüche 1 bis 5, dadurch gekennzeichnet, dass es ausserdem Hyaluronidase und $\alpha$-Glucosidase enthält.

E

Europäisches
Patentamt

EUROPÄISCHER RECHERCHENBERICHT

0162007

Nummer der Anmeldung

EP 85 81 0165

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 285 896 (NIPPON KAYAKU K.K.) <br> * Ansprüche 1,2,6,8; Seite 4, Zeilen 10-18 * <br><br> --- | 1-5 | A 61 K 37/26 <br> A 61 K 47/00 |
| X | CHEMICAL ABSTRACTS, Band 92, Nr. 22, 2. Juni 1980, Seite 295, Nr. 185837u, Columbus, Ohio, US; K. MORIMOTO et al.: "Pharmaceutical studies of polyacrylic acid aqueous gel bases: absorption of insulin from polyacrylic acid aqueous gel bases following rectal administration in alloxan diabetic rats and rabbits" & J. PHARMACOBIO-DYN. 1980, 3(1), 24-32 <br> * Zusammenfassung * <br><br> --- | 1 | |
| X | CHEMICAL ABSTRACTS, Band 98, Nr. 1, 3. Januar 1983, Seite 823, Nr. 818w, Columbus, Ohio, US; K. MORIMOTO et al.: "Effective vaginal absorption of insulin in diabetic rats and rabbits using polyacrylic acid aqueous gel bases" & INT. J. PHARM. 1982, 12(2-3), 107-11 <br> * Zusammenfassung * <br><br> ----- | 1 | RECHERCHIERTE SACHGEBIETE (Int. Cl.4) <br><br> A 61 K |

Der vorliegende Recherchenbericht wurde für alle Patentansprüche erstellt.

| Recherchenort <br> DEN HAAG | Abschlußdatum der Recherche <br> 23-07-1985 | Prüfer <br> PEETERS J.C. |
|---|---|---|